# EUROPEAN PATENT APPLICATION

(11) **EP 4 768 061 A1**
(43) Date of publication of application: **01.07.2026**
(21) Application number: 24856736.4
(22) Date of filing: 14.08.2024
(51) Int. Cl.: A61M 25/00, A61M 1/16, A61M 60/38, A61M 25/01

(54) **CATHETER**

(30) Priority: 21.08.2023 KR 20230108807; 13.06.2024 KR 20240076803
(71) Applicant: Korea University Research and Business Foundation, Seoul 02841 (KR)
(72) Inventor: YU, Cheol Woong, Seoul 03003 (KR)
(74) Representative: De Clercq & Partners
(86) International application number: PCT/KR2024/012109
(87) International publication number: WO 2025/042124

(57) **Abstract**

A catheter according to an embodiment is disclosed. In a catheter connected to an extracorporeal membrane oxygenation device, the catheter may comprise: a catheter body; and a wire which guides the intravascular insertion of the catheter body, wherein the catheter body may have, at an end portion thereof, an adjustable means for controlling a direction of outflow from the catheter body, and the adjustable means may be provided to form the end portion of the catheter body into a U-shape.

## Description

### TECHNICAL FIELD

The disclosure generally relates to a catheter, and for example, to a catheter connected to an extracorporeal membrane oxygenation device.

### BACKGROUND ART

The heart is a muscular organ located on the left side of the chest and is an essential organ for sustaining life. The heart consists of four chambers, including the two atria positioned at the upper side and the two ventricles positioned at the lower side. The valves are present between the atria and the ventricles to prevent blood from flowing backward. The atria and the ventricles are separated into the left and right sides so that blood does not mix, and are divided into the left atrium, the left ventricle, the right atrium, and the right ventricle.

The heart circulates blood throughout the entire body through contraction and relaxation. Venous blood, which has supplied oxygen to the entire body and has a reduced oxygen level, enters the right atrium and is delivered to the pulmonary artery through the right ventricle. Blood that has received sufficient oxygen supply in the lungs enters the left atrium and the left ventricle through pulmonary veins and is supplied to the entire body through the aorta.

In order to assist the functions of the heart and the lungs, an extracorporeal membrane oxygenation device may be used. The extracorporeal membrane oxygenation device is referred to as extra-corporeal membrane oxygenation and is mainly called ECMO. The extracorporeal membrane oxygenation device is a mechanical device for temporarily assisting functions of the heart and the lungs in a patient for whom sustaining life is difficult by conventional methods due to degradation of heart or lung function, such as a patient with severe cardiopulmonary failure who does not respond to optimal palliative treatment.

For example, Korean Registered Patent Publication No. KR 101634087B1 discloses an apparatus and a method for controllably assisting mitral valve motion.

The above description has been possessed or acquired by the inventor(s) in the course of conceiving the present disclosure and is not necessarily an art publicly known before the present application is filed.

### DISCLOSURE OF THE INVENTION

### TECHNICAL GOALS

An object according to an embodiment is to provide a catheter for assisting the left ventricle of a patient.

An object according to an embodiment is to provide a catheter capable of strengthening the flow of blood moving from the left ventricle to the aorta.

An object according to an embodiment is to provide a catheter for assisting a heart function essential for sustaining life by circulating blood throughout the entire body.

An object according to an embodiment is to provide a catheter that is inserted into a blood vessel and connected to an extracorporeal membrane oxygenation device to supply oxygen-treated blood to a patient.

An object according to an embodiment is to provide a catheter for overcoming Harlequin syndrome and reducing the left ventricular load.

### TECHNICAL SOLUTIONS

According to an embodiment, a catheter connected to an extracorporeal membrane oxygenation device may include a catheter body, and a wire configured to guide intravascular insertion of the catheter body, wherein an adjustable means configured to control a direction of blood outflow from the catheter body may be provided at an end portion of the catheter body, and the adjustable means may be configured to form the end portion of the catheter body into a U-shape.

In an embodiment, one end of the adjustable means may be arranged in parallel with a direction toward which an outlet of the end portion of the catheter body faces, and the other end of the adjustable means may be arranged to face a direction opposite to the direction toward which the outlet faces.

The adjustable means may be configured to change in shape from a linear shape to a U-shape after being inserted into a blood vessel.

The adjustable means may include a shape memory alloy.

The adjustable means may be guided toward an ascending aorta side by the wire, and may be configured to change a direction of the generated blood flow in accordance with a direction of blood flow exiting from the left ventricle.

The adjustable means may be disposed in an ascending aorta.

The extracorporeal membrane oxygenation device may be disposed at the other end portion of the catheter, and the generated blood flow may be formed from the extracorporeal membrane oxygenation device toward the adjustable means.

### EFFECTS OF THE INVENTION

According to an embodiment, the catheter may assist the left ventricle of a patient.

According to an embodiment, the catheter may strengthen a flow of blood moving from the left ventricle to the aorta.

According to an embodiment, the catheter may assist a heart function essential for sustaining life by circulating blood throughout the entire body.

According to an embodiment, the catheter may be inserted into a blood vessel and connected to an extracorporeal membrane oxygenation device to supply oxygen-treated blood to a patient.

According to an embodiment, the catheter may overcome Harlequin syndrome and reduce the left ventricular afterload.

Effects of the catheter according to an embodiment are not limited to those mentioned above, and other effects not mentioned will be clearly understood by those skilled in the art from the description below.

### BRIEF DESCRIPTION OF DRAWINGS

The above-described and other aspects, features, and advantages of certain embodiments of the disclosure will become apparent from the following detailed description taken in conjunction with the accompanying drawings.
FIG. 1 illustrates a first use state of a catheter according to an embodiment.
FIG. 2 illustrates a second use state of a catheter according to an embodiment.

### BEST MODE FOR CARRYING OUT THE INVENTION

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the technical scope of the disclosure.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

In addition, when describing the examples with reference to the accompanying drawings, like reference numerals refer to like elements and any repeated description related thereto will be omitted. In addition, the description of embodiments, detailed description of well-known related structures or functions will be omitted when it is deemed that such description will cause ambiguous interpretation of the disclosure.

In addition, the terms first, second, A, B, (a), and (b) may be used to describe components of the embodiments. These terms are used only for the purpose of discriminating one component from another component, and the nature, the sequences, or the orders of the components are not limited by the terms. It should be noted that if one component is described as being "connected," "coupled" or "joined" to another component, the former may be directly "connected," "coupled," and "joined" to the latter or "connected", "coupled", and "joined" to the latter via another component.

A component, which has the same common function as a component included in any one embodiment, will be described by using the same name in other embodiments. Unless disclosed to the contrary, the description of any one embodiment may be applied to other embodiments, and the specific description of the repeated configuration will be omitted.

According to an embodiment, a catheter 10 may strengthen a flow of blood moving from the left ventricle to the aorta. After gas exchange occurs in pulmonary capillaries, oxygenated arterial blood is received by the left atrium through pulmonary veins, and then the left ventricle receives the arterial blood from the left atrium and may move the blood to the aorta through contraction of the left ventricle. Here, the catheter 10, according to an embodiment, may be disposed at an external inlet of the left ventricle in the aorta (e.g., the ascending aorta), and may discharge oxygenated blood supplied from an extracorporeal membrane oxygenation device 200.

For example, an adjustable means 110 of the catheter 10, according to an embodiment, may be positioned in the ascending aorta. When blood flow is sent in a antegrade direction by the adjustable means 110, negative pressure may be formed between an end portion of the ascending aorta and the left ventricle, and due to such negative pressure, the afterload of the left ventricle may be reduced.

In this manner, a flow of oxygenated blood from the extracorporeal membrane oxygenation device 200 may be added to a flow of arterial blood moving toward the aorta by the catheter 10 according to an embodiment. Such blood flow may spread throughout the entire body through the aorta so that oxygenated blood is supplied to the entire body. Thereafter, arterial blood is converted into venous blood within the entire body, and the venous blood moves to the superior vena cava and the inferior vena cava so that the right atrium receives the venous blood, the right ventricle receives the venous blood from the right atrium, and the venous blood may be moved to the pulmonary artery through contraction of the right ventricle.

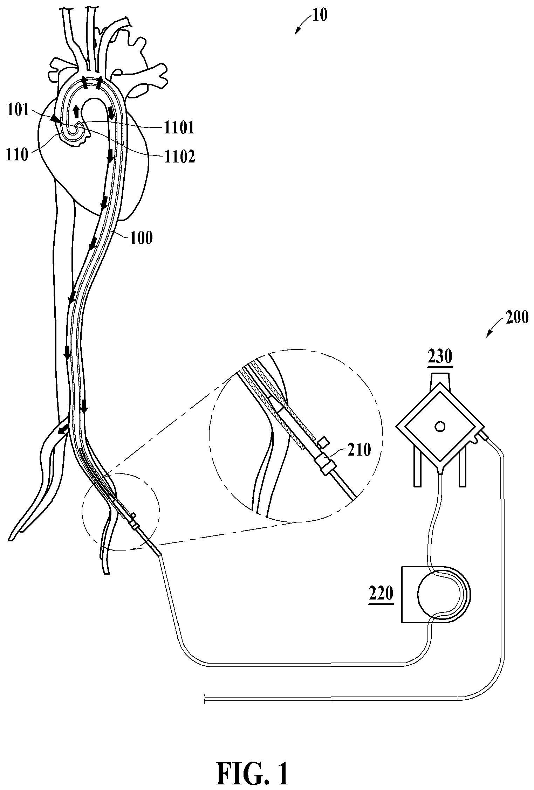

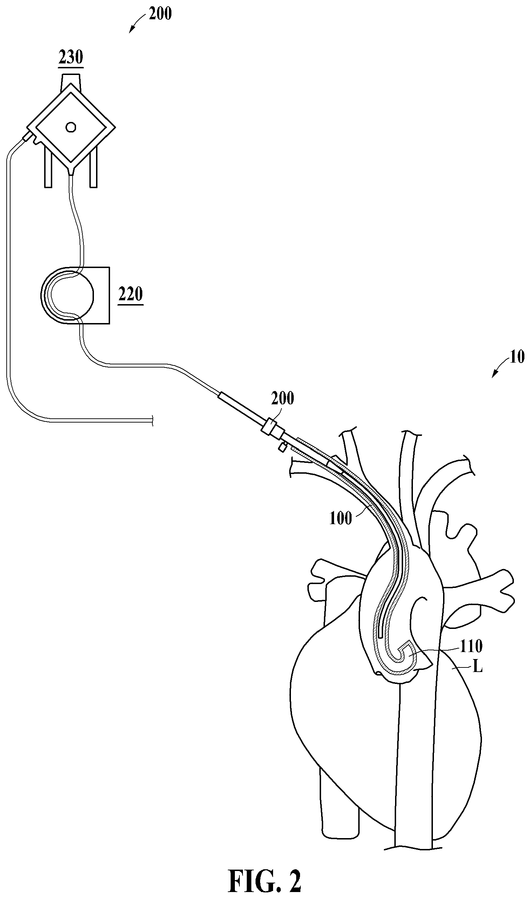

FIG. 1 illustrates a first use state of the catheter 10 according to an embodiment. FIG. 2 illustrates a second use state of the catheter 10 according to an embodiment. The catheter 10, according to an embodiment, may be connected to the extracorporeal membrane oxygenation device 200. The use state of the catheter 10 according to an embodiment is not limited to those shown in the drawings of the disclosure.

Referring to FIGS. 1 and 2, the catheter 10 may include a catheter body 100, and a wire (not shown) configured to guide intravascular insertion of the catheter body 100. For example, the catheter body 100 may be formed of a material having excellent biocompatibility, such as natural latex, polyvinyl chloride (PVC) resin, or thermoplastic polyurethane (TPU).

An adjustable means 110 configured to control a direction of blood outflow from the catheter body 100 may be provided at an end portion 101 of the catheter body 100, and the adjustable means 110 may be configured to form the end portion 101 of the catheter body 100 into a U-shape (or a C-shape).

One end 1101 of the adjustable means 110 may be arranged in parallel with a direction toward which an outlet of the end portion 101 of the catheter body 100 faces. The other end 1102 of the adjustable means 110 may be arranged to face a direction opposite to the direction toward which the outlet faces.

Here, when the adjustable means 110 controls the direction of blood outflow from the catheter body 100, the shape of the adjustable means 110 may be deformed such that the one end 1101 and the other end 1102 of the adjustable means 110 become parallel to each other, thereby converting the end portion 101 of the catheter body 100 into a U-shape.

In this manner, the adjustable means 110 may be configured to change in shape from a linear shape to a U-shape after being inserted into a blood vessel.

For example, the adjustable means 110 may include a shape memory alloy. For example, when the adjustable means 110 reaches a target location to be converted into a U-shape, such as the left ventricle side, the adjustable means 110 may reach a predetermined temperature by applying current through the catheter body 100 so that the adjustable means 110 is converted into a U-shape.

In an embodiment, the adjustable means 110 may further include a shape memory alloy adjuster.

For example, the shape memory alloy adjuster may be formed as a shape memory alloy dilator. The shape memory alloy dilator may move together with the adjustable means 110 toward the ascending aorta while allowing the adjustable means 110 to maintain a shape prior to the U-shape. When the shape memory alloy dilator is removed from the end portion 101 of the catheter body 100, the adjustable means 110 may be converted into a U-shape.

In another example, the shape memory alloy adjuster may be formed as an adjustment lever. The adjustment lever may be positioned at the other end portion of the catheter body 100 and may convert the shape of the adjustable means 110 into a U-shape.

The catheter 10 connected to the extracorporeal membrane oxygenation device 200 may receive oxygenated blood from the extracorporeal membrane oxygenation device 200, and the catheter 10 may discharge the supplied oxygenated blood in accordance with a direction of blood flow flowing from the left ventricle to the aorta to form a generated blood flow.

The adjustable means 110 may be guided toward the left ventricle side by the wire. After the adjustable means 110 is guided toward the left ventricle side, the adjustable means 110 may be configured to change a direction of the generated blood flow in accordance with a direction of blood flow exiting from the left ventricle.

Here, the adjustable means 110 may be disposed outside the left ventricle (e.g., the ascending aorta). The adjustable means 110 may be disposed near the exterior of the left ventricle within the aorta and may be converted into a U-shape.

In an embodiment, the catheter body 100 may include a side opening. Blood flow exiting through the side opening may assist forward blood flow.

The extracorporeal membrane oxygenation device 200 may be disposed at the other end portion of the catheter 10, and at least a portion of the extracorporeal membrane oxygenation device 200 may be connected thereto. At this time, a generated blood flow may be formed from the extracorporeal membrane oxygenation device toward the adjustable means 110.

At least a portion of the extracorporeal membrane oxygenation device 200 may be connected to the other end portion of the catheter 10. For example, a catheter connection portion 210 of the extracorporeal membrane oxygenation device 200 may be connected to the other end portion of the catheter 10.

The extracorporeal membrane oxygenation device 200 may include a series of devices, such as a membrane oxygenator 230 configured to supply oxygen to blood in place of the lungs, a centrifugal blood pump 220 configured to drive a device that returns oxygenated blood to the heart, and circuits configured to control oxygen and carbon dioxide.

The extracorporeal membrane oxygenation device 200 may drain blood through a drainage catheter inserted into the body, remove carbon dioxide and supply oxygen through the membrane oxygenator 230 during extracorporeal circulation, and then reinfuse oxygenated blood through a return catheter to recirculate the blood. The extracorporeal membrane oxygenation device 200 may allow blood to be drained into the body through the drainage catheter inserted into the body via the centrifugal blood pump 220.

The catheter 10, according to an embodiment, may be inserted and used at a portion where a large blood vessel is present. Depending on a type of the extracorporeal membrane oxygenation device 200, an insertion route of the catheter 10 according to an embodiment through a blood vessel may vary, and for example, selection of a blood vessel location may vary, such as the shoulder, the neck, or the thigh.

The extracorporeal membrane oxygenation device 200 may operate in a manner in which blood is withdrawn from a patient through a vein, carbon dioxide and waste materials are removed from the blood and oxygen is supplied through the membrane oxygenator 230, and then the blood is returned to the patient through a vein or an artery. A method in which blood is withdrawn from a vein and returned to an artery is referred to as venoarterial extracorporeal membrane oxygenation (VA-ECMO), and a method in which blood withdrawn from a vein is returned to a vein is referred to as venovenous extracorporeal membrane oxygenation (VV-ECMO).

VA-ECMO may be mainly used for a patient with heart failure. In the case of VA-ECMO, the catheter 10, according to an embodiment, may be inserted into an aorta (a coronary artery or an esophageal artery) and a vena cava. When the catheter 10, according to an embodiment, configured to discharge blood is positioned in a vein and drained blood passes through the oxygenator, blood with increased oxygen content may be supplied to an artery through the catheter 10 according to an embodiment.

In the case of VA-ECMO, the catheter 10, according to an embodiment, may perform a function of simultaneously assisting heart and lung functions.

VV-ECMO may be used for a patient with respiratory failure. In the case of VV-ECMO, the catheter 10, according to an embodiment, may be inserted mainly into a vena cava or an inferior vena cava. When the catheter 10, according to an embodiment, configured to discharge blood is positioned in a vein and drained blood passes through the oxygenator, blood with increased oxygen content may re-enter the vein through the catheter 10 according to an embodiment.

In the case of VV-ECMO, the catheter 10, according to an embodiment, may perform a function of assisting lung function.

Hereinafter, use states of the catheter 10 according to an embodiment will be described with reference to FIGS. 1 and 2.

Blood is moved to the aorta connected to the left ventricle through contraction of the left ventricle. The aorta sequentially extends from the left ventricle to the ascending aorta, the aortic arch, the descending thoracic aorta, and the abdominal aorta, and then branches into two common iliac arteries in the lower abdomen. The common iliac arteries extend to the femoral arteries.

In particular, referring to FIG. 1, in a first use state of the catheter 10 according to an embodiment, the catheter 10 may be inserted into a femoral artery. At this time, an end portion of the catheter body 100 is in a linear state. Thereafter, the catheter 10, according to an embodiment, may move to the aortic arch and the ascending aorta through the wire. Thereafter, the catheter 10 reaches the ascending aorta beyond the aortic arch, and then the end portion of the catheter body 100 may be converted into a U-shape through the adjustable means 110.

In particular, referring to FIG. 2, in a second use state of the catheter 10 according to an embodiment, the catheter 10 may be inserted into a blood vessel branching from the aortic arch toward the brain and the bilateral upper limbs. At this time, the end portion of the catheter body 100 is in a linear state. Thereafter, the catheter 10, according to an embodiment, may move to the aortic arch and the ascending aorta through the wire. Thereafter, the catheter 10 reaches the ascending aorta beyond the aortic arch, and then the end portion of the catheter body 100 may be converted into a U-shape through the adjustable means 110.

In this manner, after the catheter 10 according to an embodiment reaches a specific location such as a blood discharge position of the catheter 10, by performing an operation such as causing current to flow to the adjustable means 110 of the catheter 10, the shape memory alloy of the adjustable means 110 reaches a predetermined temperature and is deformed, thereby converting the end portion of the catheter body 100 into a U-shape by the adjustable means 110.

For example, the catheter 10, according to an embodiment, may be disposed such that the end portion of the catheter body 100 is positioned in the ascending aorta. In this manner, the catheter 10, according to an embodiment, may supply oxygenated blood into the ascending aorta, thereby significantly reducing the possibility of the occurrence of Harlequin syndrome. Harlequin syndrome is a complication of venoarterial extracorporeal membrane oxygenation (VA-ECMO) that may occur when the left ventricular function begins to recover. If the end portion of the catheter body 100 is maintained without being converted into a U-shape, blood with low oxygen saturation may be incorrectly delivered to an upper limb or a lower limb, thereby causing Harlequin syndrome. The catheter 10, according to an embodiment, may deliver blood with high oxygen saturation to an upper limb or a lower limb, thereby effectively preventing Harlequin syndrome.

The catheter 10, according to an embodiment, may be disposed in a pulmonary vein through which blood moves to the left ventricle so that right radial hypoxemia does not occur.

The catheter 10, according to an embodiment, may allow treatment even when there is malformation or deformation of an artery or a vein (arteriosclerosis, occlusion, and the like).

The catheter 10, according to an embodiment, may be expected to reduce the risk of occurrence of thromboembolism and may maintain smooth blood supply even to an artery below an insertion site. The catheter 10, according to an embodiment, may prevent ischemia and necrosis in a lower limb, thereby reducing pseudoaneurysm, arteriovenous fistula, and arteriovenous occlusion.

The catheter 10, according to an embodiment, may reduce the use of anticoagulants, thereby reducing hemorrhagic complications such as cerebral hemorrhage and intra-abdominal hemorrhage.

The catheter 10, according to an embodiment, may be expected to reduce complications such as pulmonary edema and pulmonary hemorrhage by improving a situation in which the lungs cannot saturate oxygen due to severe pulmonary edema or pneumonia.

An order of the use methods described herein is not limited thereto and may be performed in another order, and at least one of the described operations may be omitted, or additional operations may be included before or after any of the described operations.

While the embodiments have been described with reference to the limited drawings, it will be apparent to one of ordinary skill in the art that various alterations and modifications can be made from the above description. For example, suitable results may be achieved if the described techniques are performed in a different order, and/or if components in a described system, structure, device, or circuit are combined in a different manner, and/or replaced or supplemented by other components or their equivalents.

Therefore, other implementations, other embodiments, and/or equivalents of the claims are within the scope of the following claims.

## Claims

1. A catheter connected to an extracorporeal membrane oxygenation device, the catheter comprising:
a catheter body; and
a wire configured to guide intravascular insertion of the catheter body,
wherein an adjustable means configured to control a direction of blood outflow from the catheter body is provided at an end portion of the catheter body, and
the adjustable means is configured to bend the end portion of the catheter body.

2. The catheter of claim 1,
wherein the adjustable means is configured to form the end portion of the catheter body into a U-shape.

3. The catheter of claim 2,
wherein the catheter body comprises a side opening.

4. The catheter of claim 2,
wherein one end of the adjustable means is arranged in parallel with a direction toward which an outlet of the end portion of the catheter body faces, and the other end of the adjustable means is arranged to face a direction opposite to the direction toward which the outlet faces.

5. The catheter of claim 4,
wherein the adjustable means is configured to change in shape from a linear shape to a U-shape after being inserted into a blood vessel.

6. The catheter of claim 5,
wherein the adjustable means comprises a shape memory alloy.

7. The catheter of claim 6,
wherein the adjustable means further comprises the shape memory alloy adjuster.

8. The catheter of claim 2,
wherein the adjustable means is guided toward an ascending aorta side by the wire, and the adjustable means is configured to change a direction of a generated blood flow in accordance with a direction of blood flow exiting from a left ventricle.

9. The catheter of claim 8,
wherein the adjustable means is disposed in an ascending aorta.

10. The catheter of claim 2,
wherein the extracorporeal membrane oxygenation device is disposed at the other end portion of the catheter, and a generated blood flow is formed from the extracorporeal membrane oxygenation device toward the adjustable means.
